Europäisches Patentamt

European Patent Office

Office européen des brevets

(11) Publication number : **0 574 248 A2**

# EUROPEAN PATENT APPLICATION

(21) Application number : **93304483.6**

(51) Int. Cl.$^5$ : **A61L 15/00,** C08F 8/00,
C08J 3/24

(22) Date of filing : **09.06.93**

(30) Priority : **10.06.92 JP 150575/92**

(43) Date of publication of application :
**15.12.93 Bulletin 93/50**

(84) Designated Contracting States :
**DE FR GB IT SE**

(71) Applicant : **NIPPON SHOKUBAI CO., LTD.**
**1-1, Koraibashi, 4-chome**
**Chuo-ku, Osaka-shi, Osaka 541 (JP)**

(72) Inventor : **Hatsuda, Takumi**
**Hamada Shataku G-306, 931-11 Hamada,**
**Aboshi-ku**
**Himeji-shi, Hyogo-ken (JP)**
Inventor : **Irie, Yoshio**
**3253-3 Funatsu-cho**
**Himeji-shi, Hyogo-ken (JP)**

(74) Representative : **Rees, David Christopher et al**
**Kilburn & Strode 30 John Street**
**London WC1N 2DD (GB)**

(54) **Method for production of absorbent resin and absorbent.**

(57)   A method for production of an absorbent resin which comprises exerting a shearing force on a hydrated gel polymer having a cross-linked structure thereby obtaining a particulate hydrated gel polymer having a moisture content in the range of from 30 to 90% by weight, a polymerization ratio in the range of from 60 to 99.9% by weight, and an average particle diameter in a dry state in the range of from 100 to 1,000 $\mu$m and drying said particulate hydrated gel polymer thereby lowering said moisture content to 20% by weight or less and enhancing the polymerization ratio of said polymer to a level exceeding the level which existed before said drying, and a method for production of an absorbent which comprises mixing the absorbent resin mentioned above with a cross-linking agent having at least two functional groups capable of reacting with the functional group of said absorbent resin thereby effecting reaction of said absorbent resin with said cross-linking agent and consequently heightening the cross-linking density in the surface region of said absorbent resin. The method permits production of an absorbent resin having a high absorption rate and having only a small fine powder content. It also allows production of an absorbent resin which sparingly suffers from the occurrence of fine powder during conveyance thereof in a plant line, during transportation thereof in a distribution system, and during use in varying applications and allows the work environment to remain clean at all times.

EP 0 574 248 A2

This invention relates to a method for the production of an absorbent resin and an absorbent. More particularly, it relates to a method for the production of an absorbent resin which contains a low fine powder concentration, effects absorption at an exceptionally high rate, and is suitable as, a hygienic material for disposable diapers and sanitary articles, a water-retaining agent for agriculture, horticulture, and afforestation, and a material for a wide range of absorbent articles.

In recent years, absorbent resins have found utility in various applications covering materials for disposable diapers, water-retaining agents for agriculture and horticulture, and industrial grade dehydrators, for example. The conventional absorbent resins, however, have the disadvantage in that they have a low absorption capacity and, if this is not the case, they have a low absorption rate.

When a conventional absorbent resin of a high absorption capacity is incorporated in a disposable diaper, for example, the urine discharged therein persists on the surface of the diaper for some time before it is thoroughly absorbed by the absorbent resin layer inside the diaper. Thus, the user of this diaper is compelled to endure the unpleasant sensation of the wet skin of the diaper until the diaper becomes thoroughly dry. In these circumstances, various attempts have been made to improve the absorption rate of the absorbent resin. They have been directed, for example, to decreasing the particle diameter of the absorbent resin or producing an absorbent resin in a granular form or in the form of flakes for the purpose of increasing the surface area of the absorbent resin. Generally, when the particle diameter of the absorbent resin is decreased, however, the absorbent resin is liable to form wet clusters with the absorbed liquid in most cases at the expense of the absorption rate. When the absorbent resin is molded in a granular form, the individual granules of the resin combine into wet clusters upon absorbing liquid and mostly result in a sacrifice of the absorption rate. When the absorbent resin is molded in the form of flakes, though the absorption rate is improved, the improvement itself is not sufficient. Moreover, the production of flakes is handicapped by the extra work of shaping the flakes. The flakes of absorbent resin inevitably gain in bulkiness and, therefore, prove to be uneconomical because they require an appreciable addition to facilities for transportation and storage.

Techniques for improving the absorption rate of an absorbent resin by cross-linking the molecular chain in the surface region of the absorbent resin thereby increasing the cross-linking density in the surface layer of the absorbent resin without impairing the absorption capacity have been proposed. These techniques are disclosed in JP-B-61-48521, JP-A-58-42602, US-A-4541871, US-A-4666983, US-A-4497930, US-A-4734478, etc. These techniques have somewhat improved absorbent resins in terms of the absorption rate. Various uses are found for absorbent resins. The absorbent resin adopted for a given use is required to have a particular diameter which is optimum for the particular use. The absorbent resins which are produced by such conventional techniques, however, are in such a state that they inevitably contain a large proportion of fine powder, i.e. particles of a diameter smaller than the optimum particle diameter. The use of such absorbent resins which have a large fine powder content has the following problems.

(1) The absorbent resin, while being handled for the sake of a given use, is liable to produce dust and jeopardize the work environment and resuit in a loss of quality.

(2) The absorbent resin while absorbing liquid tends to form wet clusters and prevents the liquid from diffusing into the absorbent resin as expected and this absorbent resin, when used in a disposable diaper, tends to induce urine leakage .

(3) The ability of the absorbent resin, to mix with or disperse in another substance is poor. Thus, it is not able to form a uniform mixture with soil, for example.

(4) The absorbent resin exhibits poor flowability as a powder and, therefore, tends to form powder bridges in the tranport hopper which causes the phenomenon of flushing.

As a means for the solution of these problems, a method which pelletizes the absorbent resin in a powdery form with water alone or with water and a water-soluble polymeric compound with the aid of a binder used in combination with an inorganic powder (US-A-4734478 and JP-A-61-101536) and a method which pelletizes the absorbent resin in a powdery form by dispersing the powder in a non-aqueous liquid, adding an aqueous monomer solution to the resultant dispersion thereby causing the dispersed absorbent resin to absorb the aqueous solution, and thereafter polymerizing the resultant mixture (EP-A-233014) have been disclosed in the art.

The methods disclosed in US-A-4734478 and JP-A-61-101536, however, have the disadvantage that the pellets produced thereby have insufficient strength and these pellets, while being conveyed in a plant line or transported in a distribution system, undergo partial breakage to produce fine powder. The method disclosed in EP-A-233014 suffers from the disadvantage that the absorbent resin, while being pelletized, has difficulties absorbing the aqueous monomer solution uniformly throughout the entire volume thereof and produces pellets in a low yield and further that the produced absorbent resin has an unduly low absorption capacity because the aqueous monomer solution excessively permeates the absorbent resin powder and polymerizes therein.

Particularly when the absorbent resin is used in a sanitary material or in the field of foodstuffs, the residual monomer persisting in the absorbent resin causes anxiety from the standpoint of safety. Thus, various attempts have been made to lower the residual monomer content in the absorbent resin. For example, methods for lowering the residual monomer content by adding to the residual monomer such compounds as ammonia and amines (JP-B-33-2646 and JP-A-50-40689) and such compounds as sulfites and hydrogen sulfites (US-A-2960486 and US-A-4306955); methods for lowering the residual monomer content by polymerizing the residual monomer with a low temperature decomposition type and high temperature decomposition type initiators (JP-B-50-44280, JP-A-59-133205, and JP-A-53-141388), and methods for lowering the residual monomer content by decomposing the residual monomer with microorganisms have been proposed.

Indeed the addition of ammonia, an amine, a sulfite, or a hydrogen sulfite is effective in lowering the residual monomer content, but this effect is not obtained when the amount of the compound to be added is small. Further, the compound to be added, itself poses a problem of safety. The method which resorts to additional use of an initiator is not sufficiently effective in lowering the residual monomer content. The method which relies on the use of a microorganism has the problem of poor commercial feasibility.

US-A-4920202 proposes a method for the production of a polymer of a low residual monomer content by exposing a hydrated gel polymer to a gas having a high dew point thereby drying the polymer. This method is effective in obtaining a polymer of a low residual monomer content. When this method is operated to attain a very low residual monomer content, it has low productivity and difficult to commerciallize.

An object of this invention, therefore, is to provide a method for the production of an absorbent resin and an absorbent which have a sufficiently high absorption capacity, and absorption rate, and having a low fine powder content.

Another object of this invention is to provide a method for production of an absorbent resin and an absorbent which have a notable decrease in the amount of fine powder entrained therein, exhibit a high particle strength both in the dry state and wet state, and produce only a small amount of fine powder.

Yet another object of this invention is to provide a method for the production of an absorbent resin containing of an exceptionally small amount of residual monomer has a high level of safety, has a high absorption rate, and has only a small fine powder content.

The objects described above are accomplished by a method for the production of an absorbent resin which comprises excerting a shearing force on a hydrated gel polymer having a cross-linked structure thereby obtaining a particulate hydrated gel polymer having a moisture content in the range of from 30 to 90% by weight, a polymerization ratio in the range of from 60 to 99.9% by weight, and an average particle diameter in a dry state in the range of from 100 to 1,000 μm and drying the particulate gel polymer thereby lowering the moisture content to a level not more than 20% by weight and simultaneously enhancing the polymerization ratio of the polymer to a level exceeding the level which existed before the drying.

The objects are also accomplished by a method for the production of an absorbent which comprises obtaining an absorbent resin by exerting a shearing force on a hydrated gel polymer having a cross-linked structure thereby obtaining a particulate hydrated gel polymer having a moisture content in the range of from 30 to 90% by weight, a polymerization ratio in the range of from 60 to 99.9% by weight, and an average particle diameter in a dry state in the range of from 100 to 1,000 μm and by drying the particulate gel polymer thereby lowering the moisture content to a level to not more than 20% by weight and simultaneously enhancing the polymerization ratio of the polymer to a level exceeding the level which existed before the drying, and mixing the absorbent resin with a crosslinking agent having at least two functional groups capable of reacting with the functional group of the absorbent resin thereby effecting reaction of the absorbent resin with the crosslinking agent and consequently heightening the crosslinking density in the surface region of the absorbent resin.

By executing the method of this invention, the production of an absorbent resin which has a high absorption rate and a small fine powder content, the properties which have never been attained in any of the conventional absorbent resins, can be attained. Further, this method permits production of an absorbent resin which sparingly suffers from the occurrence of fine powder during conveyance in a plant line, during transportation in a distribution system, or during use of a varying sort and avoids deterioration of the work environment. It also allows an absorbent resin containing no additives, having an extremely small residual monomer content, enjoying high safety to be produced at high productivity. It further allows production of an absorbent which has excellent absorption characteristics, particularly absorption rate, under pressure loaded condition. This invention, therefore, allows production of an absorbent resin which is used advantageously as a sanitary material, as a water-retaining agent for agriculture, horticulture, and afforestation, and as a material for a wide range of absorbent articles.

Fig. 1 is a cross section illustrating schematically an embodiment of a container to be used for subjecting a hydrated gel polymer to a shearing force in this invention.

Fig. 2 is a schematic cross section taken through Fig. 1 along the line II-II.

Fig. 3 is a cross section illustrating schematically another embodiment of the container to be used for subjecting a hydrated gel polymer to a shearing force in the present invention.

Fig. 4 is a schematic diagram of a device to be used for drying a hydrated gel polymer obtained by this invention.

Fig. 5 is a schematic diagram of a device to be used for measuring the amount of absorption under load by an absorbent resin which is produced by this invention.

The hydrated gel polymer to be used in this invention must possess a cross-linked structure. The hydrated gel polymer, in the dry state, possesses an ability to absorb water not less than 5 times the weight of the polymer and, on absorbing water, undergoes voluminal expansion. When the method of this invention is executed on a water-soluble hydrated gel polymer possessing no crosslinked structure, the objects of this invention cannot be satisfied.

The hydrated gel polymer of this invention is generally obtained by polymerizing a water-soluble monomer. As typical examples of the water-soluble monomer usable effectively for the polymerization, anionic monomers such as (meth)acrylic acid, maleic acid (an hydride), fumaric acid, crotonic acid, itaconic acid, 2-(meth)acryloylethane sulfonic acid, 2-(meth)acryloylpropane sulfonic acid, 2-(meth)acrylamide-2-methylpropane sulfonic acid, vinyl sulfonic acid, and styrene sulfonic acid and salts thereof; nonionic hydrophilic group-containing monomers such as (meth)acrylamide, N-substituted (meth)acrylamide, 2-hydroxyethyl (meth)acrylate, 2-hydroxypropyl (meth)acrylate, methoxy polyethylene glycol (meth)acrylate, and polyethylene glycol (meth)acrylate; and amino group-containing monomers such as N,N-dimethyl amino ethyl (meth)acrylate, N,N-dimethyl amino propyl (meth)acrylate, and N,N-dimethyl amino propyl (meth)acrylamide and quaternized products thereof may be cited.

Optionally, a comonomer may be used with this water-soluble monomer in an amount large enough to impede appreciably the hydrophilicity of the hydrated gel polymer to be produced. Examples of the comonomer are acrylic esters such as methyl (meth)acrylate, ethyl (meth)acrylate, and butyl (meth)acrylate and hydrophobic monomers such as vinyl acetate and vinyl propionate.

For the polymerization under discussion, one member or a mixture of two or more members selected from the group of monomers enumerated above can be used. In consideration of the various absorption characteristics of the absorbent resin to be finally produced, the water-soluble monomer preferably contains at least 10 mol% of one member selected from the group consisting of (meth)acrylic acid and salts thereof, 2-(meth)acryloylethane sulfonic acid and salts thereof, 2-(meth)acrylamide-2-methylpropane sulfonic acid and salts thereof, (meth)acrylamide, methoxy polyethylene glycol (meth)acrylate, N,N-dimethylamino ethyl (meth)acrylates, and quaternization products thereof. It preferably contains a (meth)acrylic acid or a salt thereof as an essential component. Most preferably, (meth)acrylic acid to be used herein has 30 to 90 mol%, preferably 40 to 80 mol%, thereof neutralized in advance with a basic substance.

The hydrated gel polymer may be either a polymer of the self-crosslinking type obtained without use of a cross-linking agent or a polymer obtained by using a cross-linking agent possessing a polymerizable unsaturated group and/or a reactive functional group in an amount such that the produced absorbent resin will acquire characteristic properties reaching desired levels. As examples of the cross-linking agent, N,N'-methylene bis-(meth)acrylamide, (poly)ethylene glycol di(meth)acrylate, glycerol tri(meth)acrylate, trimethylol propane tri-(meth)acrylate, triallyl amine, triallyl cyanurate, triallyl isocyanurate, glycidyl (meth)acrylate, (poly)ethylene glycol, diethylene glycol, (poly)glycerol, propylene glycol, diethanol amine, trimethylol propane, pentaerythritol, (poly)ethylene glycol diglycidyl ether, (poly)glycerol polyglicidyl ether, epichlorohydrin, ethylene diamine, polyethylene imine, (poly)aluminum chloride, aluminum sulfate, calcium chloride, and magnesium sulfate may be cited. In consideration of their activity, one member and a mixture of two or members selected from the group of cross-linking agents enumerated above may be used. Among other cross-linking agents cited above, N,N'-methylene bis(meth)acrylamide, (poly)ethylene glycol di(meth)acrylate, and trimethylol propane tri-(meth)acrylate prove to be preferable. The amount of such a cross-linking agent to be used is generally in the range of from 0.001 to 2 mol%, preferably from 0.01 to 1 mol%, based on the amount of the monomer component.

The polymerization initiators which are effectively usable for the polymerization of the monomer component mentioned above include inorganic peroxides such as ammonium persulfate, potassium persulfate, and hydrogen peroxide, organic peroxides such as t-butyl hydroperoxide, benzoyl peroxide, and cumene hydroperoxide, azo compounds such as 2,2'-azo-bis(N,N'-dimethyl-ene isobutyl amidine) and salts thereof, 2,2'-azo-bis(2-amidinodipropane) and salts thereof, and 4,4'-azo-bis-cyanovaleric acid, and redox type initiators obtained by the combination of such reducing agents as sulfites, hydrogen sulfites, and ascorbic acid with peroxides, for example. The amount of such a polymerization initiator to be used is generally in the range of from 0.001 to 5% by weight, preferably from 0.01 to 1% by weight. In the production of the hydrated gel polymer, the polymerization may be attained simultaneously with the formation of a graft bond and a complex by performing the polymerization of the monomer component in the presence of a hydrophilic polymeric compound such as starch, cellulose, chitin, or polyvinyl alcohol, for example.

The particulate hydrated gel polymer in this invention is obtained by subjecting the hydrated gel polymer possessing a cross-linked structure to a shearing force. Specifically, it is obtained by exerting a shearing force, generated by for example, a screw type extruding device or a kneader on the hydrated gel polymer possessing the crosslinked structure resulting from aqueous solution polymerization thereby pulverizing it into a particulate form, that is, the shape of the gel is irregular. The particulate hydrated gel polymer of the water-in-oil type obtained by the so-called reversed-phase suspension polymerization is globular particles. The hydrated gel polymer of this sort generally retains on the particle surface thereof, a dispersant such as a surfactant used in the polymerization; the absorbent resin of a tenacious form incapable of producing fine powder is not obtained as required by this invention. The particulate hydrated gel polymer obtained in this invention comprises substantially discrete particles which are not linked after the fashion of a string of beads or a cord.

The particulate hydrated gel polymer to be used in this invention has a moisture content in the range of from 30 to 90% by weight. The term "moisture content" of the hydrated gel polymer as used in this invention means the amount of water contained in the hydrated gel polymer expressed in % by weight based on the total weight of the hydrated gel polymer. The production of the particulate hydrated gel polymer having a moisture content in the range of from 30 to 90% by weight is generally attained by subjecting an aqueous solution containing a given monomer in a concentration in the range of from 10 to 70% by weight to aqueous solution polymerization performed by the well-known technique and exerting a shearing force on the resultant hydrated gel polymer thereby pulverizing it into a particulate form. When the aqueous solution of the monomer to be subjected to the aqueous solution polymerization has a monomer concentration deviating from the range mentioned above, the particulate hydrated gel polymer aimed at by this invention may be obtained by adjusting the moisture content of the hydrated gel polymer consequently produced in consequence of the aqueous solution polymerization. It is also permissible to pulverize the hydrated gel polymer into a particulate hydrated gel polymer by dint of a shearing force and then adjust the moisture content of the produced particulate hydrated gel polymer in the range mentioned above. If this moisture content is less than 30% by weight, bonding of the individual particles of the hydrated gel polymer comes weak while the polymerization ratio of the particulate hydrated gel polymer is enhanced in the drying step. As a result, the absorbent resin to be obtained acquires a high fine powder content and this absorbent resin, while in use, tends to produce fine powder. Conversely, if the moisture content exceeds 90% by weight, the particulate hydrated gel polymer is not easily dried and the individual particles of the hydrated gel polymer cohere too toughly throughout the entire volume during the step of drying and, as a result, a absorbent resin with a small fine powder content as required by this invention cannot be obtained. The moisture content is preferably in the range of from 40 to 85% by weight and more preferably from 45 to 80% by weight.

It is essential that the particulate hydrated gel polymer which is used in this invention possess a polymerization ratio in the range of from 60 to 99.9% by weight. If this polymerization ratio is less than 60% by weight, the individual particles of the hydrated gel polymer cohere too toughly throughout the entire volume thereof while the polymerization ratio of the particulate hydrated gel polymer is enhanced during the drying step. Thus, the production of an absorbent resin having a small fine powder content as required by this invention cannot be obtained. If the polymerization ratio exceeds 99.9% by weight, the individual particles of the hydrated gel polymer are weakly bonded together while the polymerization ratio of the particulate hydrated gel polymer is enhanced during the drying step. The absorbent resin which is consequently obtained acquires a high fine powder content and this absorbent resin, while in use, tends to produce fine powder. The polymerization ratio is preferably in the range of from 80 to 99.5% by weight and more preferably from 90 to 99.5% by weight.

The particulate hydrated gel polymer fit for use in this invention has an average particle diameter in the range of from 100 to 1,000 μm on dry basis. It contains substantially no particle which has a size exceeding 5 mm. The method for determining the average particle diameter on a dry basis will be described specifically hereinbelow in the introductory to the working examples. The particulate hydrated gel polymer possessing an average particle diameter in the range mentioned above is obtained by a method which comprises mechanically pulverizing and cutting the hydrated gel polymer which has been produced by the aqueous solution polymerization and having of a cross-linked structure and is undergoing further polymerization or a method of mechanically pulverizing and cutting the hydrated gel polymer after the polymerization ratio thereof has reached a level in the range of from 60 to 99.9% by weight. Though the conventional well-known method may be adopted to exert a shearing force on the hydrated gel polymer having a cross-linked structure, a special measure is required for the purpose of obtaining a particulate hydrated gel polymer having the specific average particle diameter contemplated by this invention. For example, the method which, as disclosed in USSN 865,865, comprises applying a load to the hydrated gel polymer and, at the same time, repeatedly exerting a shearing force thereon is advantageously adopted for the production of the particulate hydrated gel polymer having the specific average particle diameter of this invention. If the average particle diameter on a dry basis exceeds 1,000 μm, the produced absorbent resin has an unduly low rate of absorption presumably because the particulate hydrated gel polymer has a decreased surface area. For this absorbent resin to be used as a material for sanitary articles, for example, it necessarily requires a pulverizing step after drying and consequently the amount of fine powder which occurs is increased. It is generally difficult to decrease the average particle diameter of the particulate hydrated gel polymer below 100 μm If this decrease is somehow attained, the produced absorbent resin possibly suffers from a deterioration of physical properties such as, for example, an increase in the water-soluble component thereof, because the serious mechanical damage inflicted on the hydrated gel polymer . Preferably, the average particle diameter on a dry basis is in the range of from 150 to 600 μm.

In this invention, the particulate hydrated gel polymer having the specific average particle diameter can be efficiently obtained by applying a load to the hydrated gel polymer and, at the same time, exerting a shearing force repeatedly thereon. For example, the particulate hydrated gel polymer of this invention can be obtained efficiently by heating the hydrated gel polymer to a temperature in the range of from 40° to 110 °C, preferably from 50° to 95 °C and applying a load of 0.01 to 1.5 kg/cm$^2$, preferably 0.05 to 1.0 kg/cm$^2$, to the polymer and, at the same time, exerting a shearing force repeatedly thereon.

The devices which are effectively usable for the exertion of a shearing force on the hydrated gel polymer include devices of batchwise operation such as (mechanical) pressure kneader, internal mixer, and bambury mixer and devices of continuous operation such as extruder, meat chopper, and continuous kneader, for example.

This invention is not accomplished unless the particulate hydrated gel polymer of a specific particle size in the process of polymerization at a specific polymerization ratio is dried to lower the moisture content thereof below 20% by weight and to increase the polymerization ratio to a level exceeding the level which existed before the drying. The drying mentioned above, therefore, must be carried out under conditions capable of not only lowering the moisture content but also heightening the polymerization ratio. When this operation of further heightening the polymerization ratio is omitted, the object of this invention or the production of an absorbent resin having a small fine powder content and having sufficient high particle strength to preclude the occurrence of fine powder is not attained. For the purpose of enhancing the particle strength and maximizing the efficiency of this invention, it is preferable to promote the polymerization to an extent such that the polymerization ratio of the finally produced absorbent resin will fall in the range of from 99.95 to 100% by weight, preferably from 99.97 to 100% by weight.

In a preferred embodiment of this invention, the drying is carried out by exposing the particulate hydrated gel polymer to a gaseous substance containing at least steam and having a dew point in the range of from 50° to 100 °C, preferably from 60° to 90 °C at a temperature in the range of from 80° to 250 °C, preferably from 90° to 150 °C. By effecting the drying of the particulate hydrated gel polymer at such a high dew point as mentioned above, the polymerization ratio can be sufficiently heightened, an increase of absorption rate and a decrease of fine powder content both can be attained, and the production of an absorbent resin having an extremely small residual monomer content can be obtained with high productivity. JP-A-64-26,604 discloses a method for producing a polymer of a small residual monomer content from a hydrated gel polymer by exposing the hydrated gel polymer to a gas possessing a high dew point thereby drying the polymer. This method is effective in obtaining a polymer which has a small residual monomer content. It nevertheless has the drawback in that an attempt at decreasing the residual monomer content to an extremely low level impairs the productivity of the operation and renders the performance of this operation on a commercial scale difficult. The combination of this method with that of this invention not only solves the problem of productivity but also allows production of an absorbent resin having a high absorption ratio and a small fine powder content.

Particularly by optimizing the conditions of dew point and air current speed, the absorbent resin which contains no additives, has an residual monomer content of less than 20 ppm, and fits the step of aqueous solution polymerization, can be obtained at relatively high productivity. Such absorbent resin has not hitherto been known. This product can be attained, for example, by preparing a particulate hydrated gel polymer using a polymerization initiator in a proportion in the range of from 0.1 to 0.5% by weight based on the amount of monomer component and drying the particulate hydrated gel polymer at a temperature in the range of from 90° to 120 °C with hot air having a dew point of 70° to 100 °C, and flowing at a speed in the range of from 0.5 to 5 m/sec.

The absorbent resin which is obtained by the method of production of this invention described above can be pulverized and/or disintegrated by the conventionally well-known method of pulverization to produce a particulate absorbent resin. The method which relies on a pulverizing device such as, for example, a high-speed rotary pulverizer (pin mill or hammer mill), a screw mill (coffee mill), or a roll mill can be cited, for example.

The method for the production of a particulate absorbent resin contemplated by this invention is characterized in that the absorbent resin obtained by the method of production according to this invention is pulverized

EP 0 574 248 A2

and/or disintegrated by the use of a roll mill (roll-rotating type pulverizer). The absorbent resin which is obtained by the method of production of this invention is uniformly dried substance and can be pulverized and/or disintegrated with a roll mill without preliminarily undergoing such an extra step as removal of undried portion. Due to this feature, the absorbent resin can be produced in a particulate form having a prominently low fine powder content and a narrow particle size distribution.

The method for the production of an absorbent according to this invention is characterized by converting the absorbent resin obtained by the method of production of the invention described above into a particulate absorbent resin by pulverizing and/or disintegrating and mixing the particulate absorbent resin with a cross-linking agent having at least two functional groups capable of reacting with the functional group of the absorbent resin thereby inducing the absorbent resin to react with the cross-linking agent and consequently heightening the cross-linking density in the surface region of the absorbent resin. Due to this characteristic method, a high rate of absorption and a small fine powder content can be attained and the production of an absorbent having excellent absorption characteristics under load, particularly the rate of absorption under load, is accomplished. To be specific, these objects are accomplished by subjecting the particulate absorbent resin of this invention to a surface cross-linking treatment in accordance with the method which is disclosed in JP-A-57-44627, JP-A-58-42602, JP-B-60-18650, JP-A-58-180233, JP-A-59-62665, and JP-A-61-16903, for example.

The cross-linking agents which are usable in the present invention include compounds having in the molecular unit thereof at least two functional groups capable of reacting with the carboxyl group of the absorbent resin, for example. The cross-linking agents which are usable as the component in the present invention include polyhydric alcohols such as ethylene glycol, diethylene glycol, triethylene glycol, tetraethylene glycol, polyethylene glycol, glycerol, propylene glycol, diethanol amine, triethanol amine, polyoxy propylene, oxyethylene-oxypropylene block copolymer, pentaerythritol, and sorbitol, polyglycidyl ether compounds such as ethylene glycol diglycidyl ether, polyethylene glycol diglycidyl ether, glycerol polyglycidyl ether, diglycerol polyglycidyl ether, polyglycerol polyglycidyl ether, sorbitol polyglycidyl ether, pentaerythritol polyglycidyl ether, propylene glycol diglycidyl ether, and polypropylene glycol diglycidyl ether, haloepoxy compounds such as epichlorohydrin and $\alpha$-methylfluorohydrin, polyamine compounds such as ethylene diamine, diethylene triamine, triethylene tetramine, tetraethylene pentamine, pentaethylene hexamine, and polyethylene imine, for example. One cross-linking agent or two or more mutually unreactive cross-linking agents selected from the group mentioned above may be used.

Among other compounds mentioned above, it is particularly preferable to use at least one compound selected from the group consisting of diethylene glycol, triethylene glycol, polyethylene glycol, glycerol, polyglycerol, propylene glycol, diethanol amine, triethanol amine, polyoxy propylene, oxyethylene-oxypropylene block copolymer, sorbitan fatty acid esters, poly-oxyethylene sorbitan fatty acid esters, trimethylol propane, pentaerythritol, and sorbitol.

The proportion of the cross-linking agent to be used in this invention is in the range of from 0.01 to 30 parts by weight, preferably from 0.1 to 10 parts by weight. If the proportion exceeds 30 parts by weight, the excess is wasted without producing any economic effect and detracts from the proper cross-linking effect and decreases the absorption capacity of the produced absorbent. Conversely, if this proportion is less than 0.01 part by weight, the effect of this invention is difficult to attain.

The average particle diameter of the absorbent obtained as described above is in the range of from 150 to 1,200 $\mu$m, preferably from 200 to 800 $\mu$m.

The absorbent resin or the absorbent which is obtained by the method of this invention can be made to acquire a new function by incorporating therein a water-soluble polymeric compound, a deodorant, a perfume, a medicine, a plant growth accelerator, a fungicide, an antifungal agent, a defoaming agent, a pigment, carbon black, activated carbon, short fibers, etc.

Now, this invention will be described more specifically below with reference to working examples and controls. The scope of this invention is not limited by these examples.

The moisture content, polymerization ratio, and average particle diameter on a dry basis of the particulate hydrated gel polymer to be mentioned in the following examples have been determined by the methods to be described hereinbelow. The moisture content, polymerization ratio, and particle size distribution of the dry pulverized substance and the absorption capacity, absorption rate, and particle strength of the dry pulverized substance as an absorbent resin to be mentioned hereinbelow are numerical values resulting from determinations performed by the following methods.

(A) Average particle diameter of particulate hydrated gel polymer on dry basis

A sample, 25 g in size, of a given particulate hydrated gel polymer (solids content $\alpha$% by weight) was placed in 1200 g of an aqueous 20 % by weight sodium chloride solution and stirred for 60 minutes with a stirrer tip

7

rotated at a rate of 300 rpm. After the stirring was completed, the resultant dispersant was through into sieves (9.5 mm, 2.0 mm, 0.85 mm, 0.60 mm, 0.30 mm, and 0.075 mm in mesh size) and 6000 g of an aqueous 20 % by weight sodium chloride solution was slowly poured into the sieves to effect classification of the particulate hydrated gel polymer. The portions of the particulate hydrated gel polymer retained on the sieves as a consequence of the classification were thoroughly drained and then weighed. With respect to the meshes of the sieves, the mesh size R (100) of a sieve equivalent to 100% by weight of the solids content of the particulate hydrated gel polymer was calculated in accordance with the following formula 1. The values of R (100) found for the sieves and the portions of the particulate hydrated gel polymer retained in the respective sieves expressed in % by weight based on the total amount of the polymer were plotted on a logarithmic probability chart. From the graph consequently obtained, the particle diameter corresponding to 50% by weight was read out and reported as the average particle diameter of the particulate hydrated gel polymer on dry basis.

$$R\ (100)\ (mm)\ =\ \sqrt[3]{\frac{\alpha}{100} \cdot \frac{25}{w}} \times \gamma\ (mm) \quad (1)$$

R (100) (mm) is determined wherein R (100) is the mesh size (mm) of the sieves reduced to the particle diameter of the particulate hydrated gel polymer having a solids content of 100% by weight, w is the total weight (g) of the hydrated gel polymer in a thoroughly drained form, and $\gamma$ is the mesh size (mm) of sieves through which the hydrated gel polymer swelled with an aqueous 20% sodium chloride solution.

(B) Moisture content of particulate hydrated gel polymer

A sample, 5 g in weight, of a given particulate hydrated gel polymer was spread out on a petri dish and dried thereon at 180 °C for 120 minutes. In accordance with the following formula 2, the moisture content of the particulate hydrated gel polymer was calculated.

$$Moisture\ content\ (wt\%)\ =\ \frac{5\ (g)\ -\ B1\ (g)}{5\ (g)} \times 100 \quad (2)$$

Moisture content (wt%) is determined wherein B1 is the weight of the particulate hydrated gel polymer in a dry state.

(C) Polymerization ratio of particulate hydrated gel polymer

A sample, 0.5 g in weight, of a given particulate hydrated gel polymer was dispersed in 1,000 ml of deionized water and stirred therein for two hours. The resultant dispersion was passed through a Wattman filter paper GF/F (particle retaining capacity 0.7 μm). The filtrate was tested for the residual monomer content by liquid chromatography to find the residual monomer content of the particulate hydrated gel polymer. The polymerization ratio of the particulate hydrated gel polymer was determined in accordance with the following formula 3.

Polymerization ratio (%) of particulate hydrated gel polymer = 100 (%) - Residual monomer content (%) of particulate hydrated gel polymer (3)

(D)Particle size distribution of a dry pulverized substance

A classifying column was assembled by superposing JIS standard sieves of mesh sizes of 18.5 mesh, 30 mesh, 50 mesh, and 100 mesh on a receptacle plate. With a sample, 30 g in weight, of a given dry pulverized substance of particulate hydrated gel polymer placed on the uppermost sieve, the classifying column was shaken with a sieve vibrator for 10 minutes. Then, the portions of the dry pulverized substance of particulate hydrated gel polymer retained on the sieves were weighed and the proportions thereof were reported in terms of % by weight.

(E) Moisture content of dry pulverized substance

A sample, 5 g in weight, of a given dry pulverized substance was spread out on a petri dish and dried thereon at 180°C for 120 minutes. The moisture content of the dry pulverized substance was determined in accordance with the following formula 4.

$$Moisture\ content\ (wt\%)\ =\ \frac{5\ (g)\ -\ E1\ (g)}{5\ (g)} \times 100 \quad (4)$$

Wherein E1 is the weight of the dry pulverized substance in a dry state (g).

(F) Polymerization ratio of dry pulverized substance

To 100 ml of deionized water placed in a beaker having an inner volume of 200 ml, a sample, 1.0 g in weight, of a given dry pulverized substance was added as kept stirred to gel the whole of the deionized water. One hour thereafter, the resultant gel was shrunk by the addition of 5 ml of an aqueous phosphoric acid solution and then stirred. The dispersion of dry pulverized substance was passed through a filter paper. The filtrate was analyzed by high-speed liquid chromatography.

Separately, a calibration curve obtained by similarly analyzing standard monomer solutions of known concentrations was adopted as an external standard. In consideration of the ratio of dilution of the filtrate, the polymerization ratio of the dry pulverized substance was determined in accordance with the following formula 5.

Polymerization ratio of dry pulverized substance (%) = 100 (%) - Residual monomer content of dry pulverized substance (%)    (5)

(G) Absorption capacity of dry pulverized substance

A sample, accurately 0.2 g in weight, of the dry pulverized substance which was classified with JIS standard sieves of mesh sizes ranging from 18.5 mesh through 100 mesh was uniformly placed in a tea-bag type pouch (40 mm x 150 mm) of non-woven fabric and immersed in an aqueous 0.9% NaCl solution and left standing therein for 60 minutes. At the end of the standing, the wet sample held in the pouch was weighed. The absorption capacity was determined in accordance with the following formula 6.

$$\text{Absorption capacity (g/g)} = \frac{\text{Weight after absorption (g)} - \text{Blank (g)}}{\text{Weight of dry pulverized substance (g)}} \quad (6)$$

(H) Absorption rate of dry pulverized substance

In a beaker having an inner volume of 100 ml, 50 ml of an aqueous 0.9% NaCl solution (30 °C) and a stirrer tip were placed and stirred at a rate of 600 rpm. A sample, 2.0 g in weight, of the dry pulverized substance classified with JIS standard sieves having mesh sizes ranging from 18.5 mesh through 50 mesh was placed instantaneously in the beaker and a stop watch was started. The stop watch was stopped when the stirrer tip exposed at the central part of the flow of the NaCl solution was hidden with the swelled hydrated gel polymer. The duration of stirring indicated on the stop watch was reported as the absorption rate. The duration of stirring decreases in proportion as the absorption rate increases.

(I) Particle strength

A sample, 30.0 g in weight, of the dry pulverized substance which was classified with JIS standard sieves having mesh sizes ranging from 18.5 mesh through 50 mesh and 10 g of glass beads 5 mm in diameter were placed in a glass vial having an inner volume of 225 ml. The glass vial with the contents was set on a paint shaker and shaken for 30 minutes. The dry pulverized substance of the hydrated gel polymer was separated from the glass beads and filtered through the aforementioned sieves to find the ratio (x%) of the proportion which passed the 50-mesh sieve. The particle strength was determined in accordance with the following formula 7.

Particle strength = 100 - x    (7)

It is shown that the numeral is greater, the particle strength of the sample is stronger, and such sample generates less fine powder when it is subjected to impact.

Example 1

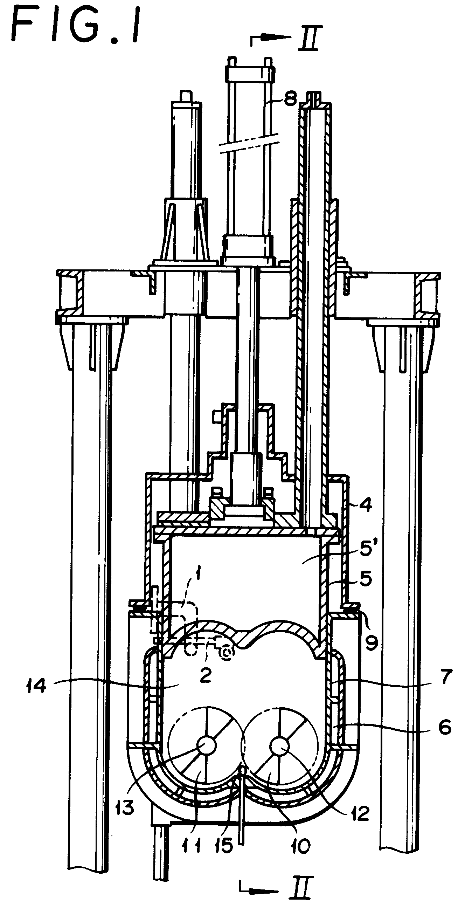

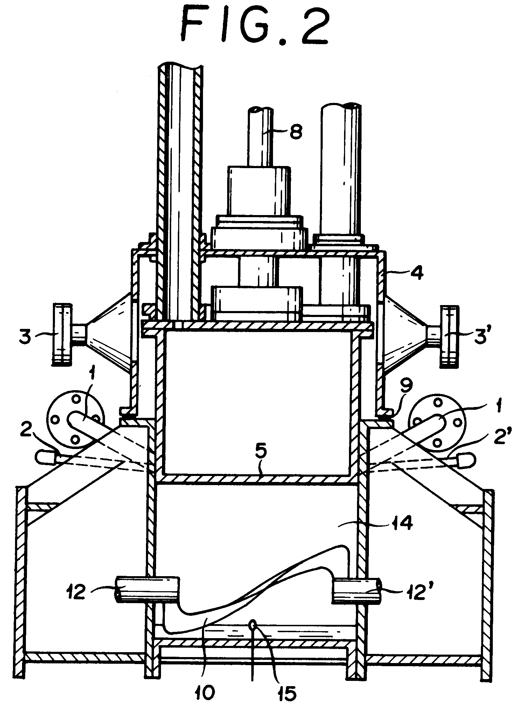

With reference to Figs. 1 and 2, a loading kneader 7 of stainless steel having a total inner volume of 75 liters and an available shear volume of 24.9 liters, provided with twin arm Z vanes and a thermometer 15, and covered with a jacket 6 was so arranged as to set a loading lid 5 and a cover 4 in the states illustrated in Fig. 1, keep a liquid inlet 1 and a nozzle 2' both in a closed state, introduce nitrogen at a flow rate of 50 liters/min. through a gas inlet 2, and discharge the gas through a gas outlet 1'. Mean while, the cover 4 was caused by its own weight to remain in tight contact with the loading kneader 7 through the medium of a sealing member 9. In the meantime, nitrogen was introduced at a flow rate of 80 liters/min through a gas inlet 3 and the gas was discharged through a gas outlet 3'. This operation was continued for 10 minutes to substitute the gas trapped in the system. Then, the gas outlet 3' was closed. In a separate vessel, nitrogen gas was blown into a mixture of 30 kg of an aqueous solution of a monomer component comprising 75 mol% of sodium acrylate and 25 mol% of acrylic acid (monomer component content 37% by weight) and 18.6 g of trimethylol propane triacrylate (0.05 mol% based on the monomer component) as a cross-linking agent to expel the dissolved oxygen from the mixture. The aqueous solution of the monomer component consequently obtained was introduced with the pressure of nitrogen into the loading kneader through the liquid inlet 1.

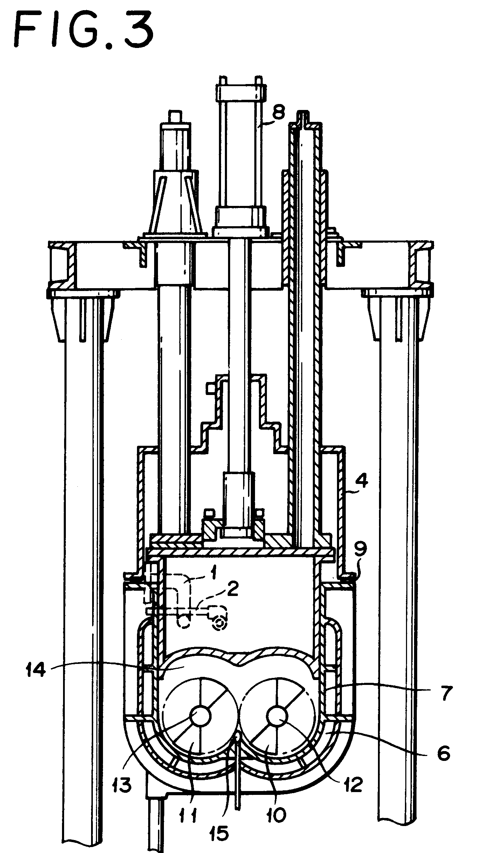

Then, the two kneader vanes were rotated at a rate of 40 rpm and water at 35 °C was passed through the jacket 6 and a jacket 5' inside the loading lid to heat the monomer component. Subsequently, an aqueous solution containing 15.0 g of sodium persulfate as a polymerization initiator and an aqueous solution containing 0.75 g of L-ascorbic acid were added to the monomer component through the liquid inlet 1. The liquid inlet 1 was closed after the addition was completed. The monomer component began to polymerize one minute after the addition of the polymerization initiator and reached the peak temperature of polymerization, 89 °C, 11 minutes thereafter. The stirring was continued at a rate of 40 rpm. After 20 minutes following the start of the polymerization, a hydrated gel polymer (A) was obtained. The temperature of the hot water in the jacket was raised to 70 °C and the vanes were rotated at 40 rpm in the hydrated gel polymer (A) adjusted at about 65 °C and, at the same time, a cylinder 8 was moved with a hydraulic device to lower the loading lid 5 until it assumed the state illustrated in Fig. 3. To be specific, the hydraulic pressure of the cylinder 8 was so adjusted as to apply a loading of 0.40 kg/cm$^2$ to the hydrated gel polymer (A) and a shearing force was exerted to the hydrated gel polymer (A) kept under a load for 20 minutes in an atmosphere of nitrogen, to obtain a hydrated gel polymer 1 in a finely divided particulate form.

The hydrated gel polymer 1 thus obtained was removed after the loading lid 5 was raised by moving the cylinder 8 with the hydraulic device and the loading kneader 7 was tilted. The polymerization ratio and moisture content of the hydrated gel polymer 1 were respectively 98.5% and 62%. In a metal net cage 200 mm x 280 mm x 80 mm, 1 kg of the hydrated gel polymer was dried with hot air at 160 °C for 30 minutes. The dried polymer was pulverized with a roll mill to afford a dry pulverized polymer 1 having a particle diameter small enough to pass a 18.5-mesh (850 μm) sieve. The polymerization ratio and moisture content of the dry pulverized polymer 1 were 99.97% and 6% respectively. The produced hydrated gel polymer was tested for average particle diameter on a dry basis and the dry pulverized polymer was tested for particle size distribution, absorption capacity, absorption rate, and particle strength as an absorbent resin by the methods described above. The results are shown in Table 1.

Control 1

A hydrated gel polymer (1a) for comparison was obtained by following the procedure of Example 1, except the loading lid 5 was not lowered so much to assume the state illustrated in Fig. 3 and the shearing force was exerted on the hydrated gel polymer (A) under no load conditions in an atmosphere of nitrogen for 10 minutes, with the loading kneader arranged in the state illustrated in Fig. 1. The polymerization ratio and moisture content of the hydrated gel polymer (1a) for comparison were respectively 98.3% and 62%. This hydrated gel polymer (1a) for comparison was dried with hot air at 160 °C for 30 minutes in the same manner as in Example 1. It, however, dried unevenly and retained an undried portion and could not be pulverized. Thus, the partly dried polymer was further dried for 30 minutes and pulverized in the same manner as in Example 1, to obtain a dry pulverized polymer(1a) for comparison. The polymerization ratio and moisture content of the dry pulverized polymer (1a) for comparison were respectively 99.96% and 6%. The hydrated gel polymer for comparison and the dry pulverized polymer for comparison were tested in the same manner as in Example 1. The results are shown in Table 1.

Control 2

The hydrated gel polymer (1a) for comparison obtained in Control 1 was dried with hot air at 160 °C for 60 minutes in the same manner as in Control 1. The resultant dry hydrated gel polymer was pulverized with a hammer mill to obtain a dry pulverized polymer (2a) for comparison having an average particle diameter of 220 μm and 30-mesh pass. The polymerization ratio and moisture content of the dry pulverized polymer (2a) for comparison were respectively 99.96% and 6%. An agglomerated polymer(2a) for comparison having a particle size of 18.5-mesh pass was obtained by mixing 100 parts by weight of the dry pulverized polymer (2a) for comparison with 5 parts by weight of water by means of a high-speed stirrer type mixing device (Turbulizer), then disintegrating and agglomerating the resultant mixture. The polymerization ratio and moisture content of the agglomerated polymer (2a) for comparison were respectively 99.96% and 10%. The agglomerated polymer (2a) for comparison thus obtained was tested in the same manner as in Example 1. The results are shown in Table 1.

Example 2

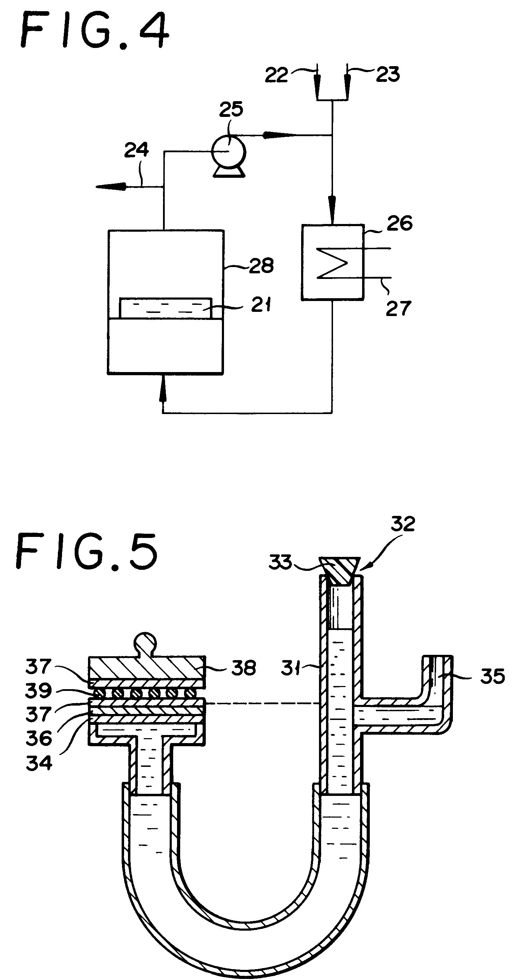

In the same loading kneader as used in Example 1, 18 kg(monomer component content 38% by weight) of an aqueous solution of a monomer component comprising of 60 mol% of sodium acrylate,10 mol% of sodium 2-methacryloyl ethanesulfonate, and 30 mol% of acrylic acid and 6.2 g (0.03 mol% based on the monomer component) of trimethylol propane triacrylate as a cross-linking agent were processed in the same manner as in Example 1 to effect polymerization of the monomer component, to produce a particulate hydrated gel polymer (B). While the hydrated gel polymer (B) was adjusted to about 65 °C by keeping the temperature of the water in the jacket at 70 °C, the vanes of the kneader were rotated at a rate of 30 rpm and the loading lid 5 was lowered so as to assume the state illustrated in Fig. 3 by moving the cylinder 8 with the hydraulic device. To be specific, the hydraulic pressure of the cylinder 8 was so adjusted as to apply a pressure of 0.12 kg/cm$^2$ on the hydrated gel polymer (B) and a shearing force was exerted on the hydrated gel polymer (B) under load in an atmosphere of nitrogen for 10 minutes, to obtain a hydrated gel polymer 2 in a finely divided particulate form. The polymerization ratio and moisture content of the hydrated gel polymer 2 were respectively 98.1% and 61%. In a hot air drier (an air current drier illustrated in Fig. 4), 1 kg of the hydrated gel polymer 2 was spread out in a thickness of 35 mm.

Then, the gases entering through a fresh air inlet and a steam inlet were introduced into a heat exchanger and heated by means of a heat medium introduced through a heat medium inlet to give rise to hot air comprising a steam-air mixture having a temperature of 105 °C and a dew point of 85 °C. When this hot air was blown at a flow rate of 0.8 m/sec. against the spread layer of the hydrated gel polymer, a dry polymer was obtained. Part of the mixed gas was discharged through an outlet and circulated with a blower to the heat exchanger. The dry polymer produced as described above was pulverized in the same manner as in Example 1 to obtain a dry pulverized polymer 2. The polymerization ratio and moisture content of the dry pulverized polymer were respectively 99.995% and 8%. The hydrated gel polymer and the dry pulverized polymer were tested in the same manner as in Example 1. The results are shown in Table 1.

Control 3

A hydrated gel polymer (3a) for comparison was obtained by following the procedure of Example 2, except that the loading lid 5 was not lowered so as to assume the state illustrated in Fig. 3 and a shearing force was exerted on the hydrated gel polymer (B) under no load conditions in an atmosphere of nitrogen for 10 minutes, with the loading kneader kept in the state illustrated in Fig. 1. The polymerization ratio and moisture content of the hydrated gel polymer (3a) for comparison were respectively 98.0% and 61%. The hydrated gel polymer (3a) for comparison was dried with hot air in the same manner as in Example 2. It, however, dried unevenly and retained an undried portion and could not be pulverized. It was further dried for 80 minutes. The dry polymer consequently obtained was pulverized in the same manner as in Example 2, to produce a dry pulverized polymer (3a) for comparison. The polymerization ratio and moisture content of the dry pulverized polymer (3a) for comparison were respectively 99.991% and 9%. The hydrated gel polymer for comparison and the dry pulverized polymer obtained herein were tested in the same manner as in Example 2. The results are shown in Table 1.

Example 3

In the same loading kneader as used in Example 1, 18 kg(monomer component content 50% by weight) of an aqueous solution of a monomer component comprising 70 mol% of potassium acrylate, 10 mol% of acrylamide, and 20 mol% of acrylic acid, 2.8 g (0.02 mol% based on the monomer component) of N,N'-methylene bis-acrylamide as a crosslinking agent, an aqueous solution of 1.8 g of sodium persulfate, and an aqueous solution of 0.1 g of L-ascorbic acid were processed to effect polymerization of the monomer component in the same manner as in Example 1, to obtain a particulate hydrated gel polymer(C). While the hydrated gel polymer (C) was adjusted to about 65 °C by keeping the temperature of the water in the jacket at 70 °C, the vanes of the kneader were rotated at a rate of 30 rpm and the loading lid 5 was lowered so as to assume the state illustrated in Fig. 3 by moving the cylinder 8 with the hydraulic device. To be specific, the hydraulic pressure of the cylinder was so adjusted as to apply a pressure of 0.12 kg/cm$^2$ to the hydrated gel polymer (C) and a shearing force was exerted on the hydrated gel polymer (C) under load for 10 minutes in an atmosphere of nitrogen, to obtain a hydrated gel polymer 3 in a finely divided particulate form. The polymerization ratio and moisture content of the hydrated gel polymer 3 were 99.0% and 46% respectively. The hydrated gel polymer thus obtained was dried with hot air and pulverized in the same manner as in Example 1, to obtain a dry pulverized polymer 3. The polymerization ratio and moisture content of the dry pulverized polymer were respectively 99.975% and 6%. The hydrated gel polymer and the dry pulverized polymer obtained herein were tested in the same manner as in Example 1. The results are shown in Table 1.

Example 4

The same loading kneader as used in Example 1 was charged with 18 kg (monomer component content 20% by weight) of an aqueous solution of a monomer component comprising 30 mol% of sodium acrylate and 70 mol% of acrylic acid and 3.5 g (0.05 mol% based on the monomer component) of N,N'-methylene bis-acrylamide as a cross-linking agent. Then, the two kneader vanes were rotated at a rate of 40 rpm and water at 35 °C was passed through the jacket 6 and the jacket 5' inside the pressure lid to heat the monomer component. Subsequently, an aqueous solution containing 5.5 g of sodium persulfate as a polymerization initiator and an aqueous solution containing 0.2 g of L-ascorbic acid were added to the contents of the loading kneader through the liquid inlet 1. The monomer component began to polymerize three minutes after the addition of the polymerization initiator. At this point, the rotation of the kneader vanes was stopped. After 17 minutes had elapsed since the stopping of the kneader vanes, the monomer component in the process of polymerization reached the peak temperature, 72 °C, of polymerization and formed a hydrated gel polymer (D). The hydrated gel polymer (D) was adjusted to about 65 °C by keeping the temperature of the water in the jacket at 70 °C and the kneader vanes were rotated at a rate of 40 rpm.

After 0.97 kg of granular sodium carbonate was added as a neutralizing agent to the pulverized hydrated gel polymer (D), the loading lid 5 was lowered so as to assume the state illustrated in Fig. 3 by moving the cylinder 8 with the hydraulic device. To be specific, the hydraulic pressure of the cylinder 8 was so adjusted as to apply a pressure of 0.12 kg/cm$^2$ to the hydrated gel polymer (D) and a shearing force was exerted on the hydrated gel polymer (D) under load in an atmosphere of nitrogen for 15 minutes, to obtain a hydrated gel polymer 4 in a finely divided particulate form. The polymerization ratio and moisture content of the hydrated gel polymer 4 were respectively 97.9% and 78%. The hydrated gel polymer 4 was dried with hot air (for a period of 60 minutes) and pulverized in the same manner as in Example 1, to obtain a dry pulverized polymer. The polymerization ratio and moisture content of the dry pulverized polymer were respectively 99.95% and 8%. The hydrated gel polymer and the dry pulverized polymer obtained herein were tested in the same manner as in Example 1. The results are shown in Table 1.

Control 4

The hydrated gel polymer (1a) obtained in Control 1 was dried with hot air at 160 °C for 60 minutes in the same manner as in Control 1. The dry polymer for comparison consequently obtained was pulverized with a hammer mill, to obtain a dry pulverized polymer having an average particle diameter of 420 μm. The addition of 60 parts by weight of water to 40 parts by weight of this dry pulverized polymer gave birth to a hydrated gel polymer (4a) for comparison having a polymerization ratio of 99.96% and a moisture content of 60%. The hydrated gel polymer(4a) for comparison was dried with hot water and pulverized in the same manner as in Example 1, to obtain a dry pulverized polymer (4a) for comparison. The polymerization ratio and moisture content of the dry pulverized polymer (4a) for comparison were respectively 99.96% and 6%. The hydrated gel polymer for comparison and the dry pulverized polymer for comparison obtained herein were tested in the same manner as in Example 1. The results are shown in Table 1.

Example 5

In the same loading kneader as used in Example 1, 18 kg (monomer component content 38% by weight) of an aqueous solution of a monomer component comprising 75 mol% of sodium acrylate and 25 mol% of acrylic acid, 6.9 g (0.03 mol% based on the monomer component) of trimethylol propane triacrylate as a cross-linking agent, an aqueous solution of 21.7g of sodium persulfate, and an aqueous solution of 0.4 g of L-ascorbic acid were processed to effect polymerization of the monomer component in the same manner as in Example 1, to obtain a particulate hydrated gel polymer (E). While the hydrated gel polymer (E) was adjusted to about 65 °C by keeping the temperature of the water in the jacket at 70 °C, the kneader vanes were rotated at a rate of 30 rpm and the loading lid 5 was lowered so as to assume the state illustrated in Fig. 3 by moving the cylinder 8 with the hydraulic device. To be specific, the hydraulic pressure of the cylinder 8 was so adjusted as to apply a pressure of 0.12 kg/cm$^2$ to the hydrated gel polymer (E) and a shearing force was exerted on the hydrated gel polymer (E) under load in an atmosphere of nitrogen for 10 minutes, to obtain a hydrated gel polymer 5 in a finely divided particulate form. The polymerization ratio and moisture content of the hydrated gel polymer were respectively 99.1% and 61%. The hydrated gel polymer 5 thus obtained was dried and pulverized in the same manner as in Example 2, to obtain a dry pulverized polymer 5. The polymerization ratio and moisture content of the dry pulverized polymer 5 were respectively 99.999% and 8%. The hydrated gel polymer and the dry pulverized polymer obtained herein were tested in the same manner as in Example 1. The results are shown in Table 1.

Example 6

One hundred (100) parts by weight of the dry pulverized polymer (1) obtained in Example 1 was mixed with an aqueous mixture comprising 1 part by weight of glycerol, 2 parts by weight of water, and 4 parts by weight of isopropanol. The produced mixture was thrown in a bowl immersed in an oil bath(195 °C) and stirred and heat-treated therein for 35 minutes, to obtain an absorbent 6. The absorbent thus obtained was tested in the same manner as in Example 1. The results are shown in Table 1. It was tested for absorption properties under load in accordance with the method described below. The absorption capacities under load applied for 5 minutes and 30 minutes were respectively 17 ml/g and 27 ml/g.

Method for evaluation of absorption properties under load

By the use of an apparatus illustrated in Fig. 5, the absorbent was tested for volumes of absorption under load applied for 5 minutes and 30 minutes and evaluated for absorption rate under load and for absorption capacity under load. A stopper 33 was fit in an upper mouth 32 of a buret 31 and a measuring table 34 and an air inlet 35 were set at an equal height. On a glass filter (No. 1) 36 having a diameter of 70 mm and placed in the measuring table 34, a filter paper, 0.2 g of the absorbent, and a filter paper 37 were superposed and a weight of 20 g/cm$^2$ was mounted thereon. The volume of synthetic urine (composed of 1.9% of urea, 0.8% of NaCl, 0.1% of $CaCl_2$, and 0.1% of $MgSO_4$) absorbed by the absorbent during a stated period of time was reported as the absorption capacity (ml/g) under load.

Control 5

An absorbent (5a) for comparison was obtained by following the procedure of Example 6, except that the dry pulverized polymer (1a)for comparison obtained in Control 1 was used in the place of the dry pulverized polymer (1). The produced absorbent was evaluated in the same manner as in Example 1. The results are shown in Table 1. When this absorbent was tested for absorption properties under load in the same manner as in Example 6, the absorption capacities under load applied for 5 minutes and 30 minutes were respectively 11 ml/g and 25 ml/g.

Table 1

| | Example 1 | Control 1 | Control 2 | Example 2 | Control 3 | Example 3 | Example 4 | Control 4 | Example 5 | Example 6 | Control 5 |
|---|---|---|---|---|---|---|---|---|---|---|---|
| Hydrated gel polymer | (1) | (1a) | Dry pulverized substance | (2) | (3a) | (3) | (4) | (4a) | (5) | | |
| Average particle diameter on dry basis ($\mu$m) | 220 | 1500 | 220 | 550 | 2000 | 380 | 300 | 420 | 530 | | |
| Dry pulverized substance | (1) | (1a) | Agglomerated polymer for comparison (2a) | (2) | (3a) | (3) | (4) | (4a) | (5) | Absorbent (6) | Absorbent for comparison (5a) |
| Particle size distribution (%) | | | | | | | | | | | |
| 850 $\mu$m on | 0 | 3 | 0 | 0 | 4 | 0 | 0 | 2 | 0 | 0 | 4 |
| 600 $\mu$m on | 6 | 10 | 23 | 7 | 12 | 7 | 8 | 13 | 8 | 9 | 13 |
| 300 $\mu$m on | 60 | 38 | 44 | 54 | 32 | 57 | 53 | 35 | 56 | 65 | 41 |
| 150 $\mu$m on | 28 | 34 | 22 | 31 | 36 | 29 | 31 | 34 | 29 | 22 | 32 |
| 150 $\mu$m thru | 6 | 15 | 11 | 8 | 16 | 7 | 8 | 16 | 7 | 4 | 10 |
| Absorption capacity (g/g) | 50 | 45 | 44 | 56 | 51 | 46 | 48 | 45 | 56 | 46 | 41 |
| Absorption rate (sec.) | 52 | 92 | 80 | 58 | 86 | 60 | 66 | 90 | 54 | 50 | 95 |
| Agglomeration Strength | 92 | 94 | 67 | 93 | 94 | 93 | 90 | 72 | 94 | 95 | 94 |

## Claims

1.  A method for the production of an absorbent resin, which comprises exerting a shearing force on a hydrated gel polymer having a cross-linked structure thereby obtaining a particulate hydrated gel polymer having a moisture content in the range of from 30 to 90% by weight, a polymerization ratio in the range of from 60 to 99.9% by weight, and an average particle diameter in a dry state in the range of from 100 to 1,000 μm and drying said particulate hydrated gel polymer thereby lowering said moisture content to a level not more than 20% by weight and simultaneously enhancing the polymerization ratio of said polymer to a level exceeding the level which existed before said drying.

2.  A method according to claim 1, wherein said shearing force is exerted inside a kneader provided with a plurality of rotary stirring axes.

3.  A method according to claim 1, wherein said hydrated gel polymer is obtained by polymerizing a monomer component containing 10 mol% or more of at least one monomer selected from the group consisting of (meth)acrylic acid, metal salts and ammonium salt of (meth)acrylic acid, 2-(meth)acryloyl ethane sulfonic acid (and salts thereof), 2-(meth)acrylamide-2-methylpropane sulfonic acid (and salts thereof), (meth)acryl amide, methoxy polyethylene glycol (meth)acrylates, N,N-dimethylamino-ethyl (meth)acrylate and quaternized products thereof.

4.  A method according to claim 1, wherein the average particle diameter of said particulate hydrated gel polymer in a dry state is in the range of from 150 to 600 μm.

5.  A method according to claim 1, wherein said particulate hydrated gel polymer contains substantially no particulate hydrated gel polymer having a size of 5 mm or more.

6.  A method according to claim 1, wherein said drying is effected by exposing said particulate hydrated gel polymer to a gas containing at least steam, said gas having a dew point in the range of from 50° to 100 °C at a temperature in the range of from 80° to 250 °C.

7.  A method according to claim 6, wherein said particulate hydrated gel polymer has the polymerization ratio thereof enhanced to a level in the range of from 99.95 to 100% by said drying of said polymer.

8.  A method for the production of an absorbent resin, which comprises pulverizing and/or disintegrating an absorbent resin obtained by the method recited in claim 1 by the use of a roll mill (roll rotating type pulverizer).

9.  A method according to claim 1, wherein said hydrated gel polymer has a polymerization ratio in the range of from 80 to 99.5% by weight.

10. A method according to claim 1, wherein said shearing force is exerted on said hydrated gel polymer at a temperature in the range of from 40° to 110 °C under the application of a load in the range of from 0.01 to 1.5 kg/cm$^2$.

11. A method according to claim 1, wherein said drying is effected by exposure of said particulate hydrated gel polymer to a gas containing at least steam, said gas having a dew point in the range of from 60° to 90 °C at a temperature in the range of from 90° to 150 °C.

12. A method for the production of an absorbent, which comprises obtaining absorbent resin by exerting a shearing force on a hydrated gel polymer having a cross-linked structure thereby obtaining a particulate hydrated gel polymer having a moisture content in the range of from 30 to 90% by weight, a polymerization ratio in the range of from 60 to 99.9% by weight, and an average particle diameter in a dry state in the range of from 100 to 1,000 μm, and by drying said particulate hydrated gel polymer thereby lowering said moisture content to a level not more than 20% by weight and simultaneously by enhancing the polymerization ratio of said polymer to a level exceeding the level which existed before said drying, and mixing said absorbent resin with a cross-linking agent having at least two functional groups capable of reacting with the functional group of said absorbent resin thereby effecting reaction of said absorbent resin with said cross-linking agent and consequently heightening the cross-linking density in the surface region of said absorbent resin.

13. A method according to claim 12, wherein said shearing force is exerted inside a kneader provided with a plurality of rotary stirring axes.

14. A method according to claim 12, wherein said hydrated gel polymer is obtained by polymerizing a monomer component containing 10 mol% or more of at least one monomer selected from the group consisting of (meth)acrylic acid, metal salts and ammonium salt of (meth)acrylic acid, 2-(meth)acryloyl ethane sulfonic acid (and salts thereof), 2-(meth)acrylamide-2-methylpropane sulfonic acid (and salts thereof), (meth)acryl amide, methoxy polyethylene glycol (meth)acrylates, N,N-dimethylamino-ethyl (meth)acrylate and quaternized products thereof.

15. A method according to claim 12, wherein the average particle diameter of said particulate hydrated gel polymer in a dry state is in the range of from 150 to 600 $\mu$m.

16. A method according to claim 12, wherein said particulate hydrated gel polymer contains substantially no particulate hydrated gel polymer having a size of 5 mm or more.

17. A method according to claim 12, wherein said drying is effected by exposing said particulate hydrated gel polymer to a gas containing at least steam, said gas having a dew point in the range of from 50° to 100 °C at a temperature in the range of from 80° to 250 °C.

18. A method according to claim 17, wherein said particulate hydrated gel polymer has the polymerization ratio thereof enhanced to a level in the range of from 99.95 to 100% by said drying of said polymer.

# FIG.1

# FIG.2

# FIG.3

# FIG.4

# FIG.5